**Europäisches Patentamt**

**European Patent Office**

**Office européen des brevets**

(19)

(11) Veröffentlichungsnummer : **0 391 224 B1**

## (12) EUROPÄISCHE PATENTSCHRIFT

(45) Veröffentlichungstag der Patentschrift :
**20.10.93 Patentblatt 93/42**

(51) Int. Cl.[5] : **A61K 45/06, A61K 37/66**

(21) Anmeldenummer : **90105871.9**

(22) Anmeldetag : **28.03.90**

(54) **Arzneimittel und ihre Verwendung zur Behandlung von Parasitosen.**

(30) Priorität : **01.04.89 DE 3910568**

(43) Veröffentlichungstag der Anmeldung :
**10.10.90 Patentblatt 90/41**

(45) Bekanntmachung des Hinweises auf die
Patenterteilung :
**20.10.93 Patentblatt 93/42**

(84) Benannte Vertragsstaaten :
**AT BE CH DE DK ES FR GB GR IT LI LU NL SE**

(56) Entgegenhaltungen :
**JOURNAL OF IMMUNOLOGY. vol. 139, no. 10, 15
November 1987, BALTIMORE US Seiten 3493 - 3496;
CLARK I.A. et al: "Inhibition of murine malaria
(Plasmodium chabaudi) in vivo by recombinant in-
terferon-gamma or tumor necrosis factor, and its
enhancement by butylated hydroxyanisole. "**

(56) Entgegenhaltungen :
**JOURNAL OF IMMUNOLOGY. vol. 140, no. 12, 15
Juni 1988, BALTIMORE US Seiten 4342 - 4347; REED
S.G.: "In vivo administration of recombinant INF-
gamma induces macrophage activation, and prevents acute disease, immune suppression, and death in experimental Trypanosoma Cruzi infections."
SCIENCE vol. 240, no. 4351, 22 April 1988, WAS-
HINGTON Seiten 516 - 518; SUZUKI Y. ET AL:
"Interferon-gamma: the major mediator of resistance against Toxoplasma gondii."
Allgemeine und spezielle Pharmakologie und Toxikologie 1983, S. 606 Forth W. et al.
Medizinische Mikrobiologie 1986, S. 463 Wiesmann
et al.**

(73) Patentinhaber : **BOEHRINGER INGELHEIM
INTERNATIONAL GmbH
D-55216 Ingelheim (DE)**

(72) Erfinder : **Goeth, Hanns, Dr.
Oberer Bühl 6
D-7959 Biberach 1 (DE)**
Erfinder : **Frank, Werner, Prof. Dr.
Schellingstrasse 8
D-7024 Filderstadt 4 (DE)**
Erfinder : **Renner, Ingeborg, Dipl.-Biol.
Franziskaweg 6
Stuttgart 70 (DE)**

EP 0 391 224 B1

**Beschreibung**

Gegenstand der vorliegenden Erfindung sind Arzneimittelkombinationen, bestehend aus einer Wirkstoffkombination von Interferon-gamma (IFNγ) und mindestens einem Anthelminthikum, und deren Verwendung zur Behandlung von Parasitosen bei Säugetieren.

Die mit den erfindungsgemäßen Arzneimittelkombinationen zu behandelnden Erkrankungen umfassen die aufgrund eines Befalls durch Endoparasiten, besonders der Klasse Cestodes, vorzugsweise der Ordnung Cyclophyllidea, insbesondere durch die Gattung Echinococcus, verursachte Infektionen. Die erfindungsgemäßen Arzneimittelkombinationen werden beispielsweise zur Behandlung von Infektionen durch die Art Echinococcus multilocularis eingesetzt.

Besonders die alveoläre Echinokokkose kann als gefährlichste Parasitose des Menschen in Mitteleuropa angesehen werden (FRANK, W., in: Räumliche Persistenz und Diffusion von Krankheiten [W. Fricke, E. Hinz, Hrsg.], Heidelb. Geogr. Arb. 83, 86-113, 1987).

Diese Einschätzung wird auch von der Weltgesundheitsorganisation (WHO) vertreten.

Die Infektion mit Eiern des Echinococcus hat für den Fehlzwischenwirt Mensch meist katastrophale Folgen.

Das Finnenwachstum bei E. multilocularis verläuft im Menschen langsamer als in den natürlichen Zwischenwirten, Protoscoleces werden häufig gar nicht ausgebildet und zentrale Nekroseprozesse sind typisch. Die Erkrankung beeinträchtigt die menschliche Gesundheit oft so stark, daß sie zum Tode führen kann. Nach I. DROLSHAMMER et al., Schweiz. med. Wschr. 103, 1337-1386, 1973, beträgt die Mortalität nach einer mittleren Verlaufszeit von 3,7 Jahren 52% nach Diagnosestellung und nach H.J. SCHICKER, Inaugural Diss. Med. Fak. Universität Tübingen, 1976, liegt die 5-Jahres-Überlebenszeit nach Diagnosestellung bei etwa 20%.

Der Krankheitsverlauf beim Menschen ist zudem sehr heimtückisch. Denn die Diagnose wird in den allermeisten Fällen zu spät gestellt, sodaß nach den ersten Symptomen einer Echinokokkose mit Befall der Leber die Überlebenschancen nur noch gering sind und nur noch eine infauste Prognose gegeben werden kann.

In der seit der Erstbeschreibung der Echinokokkose durch Virchow in Jahre 1856 bis 1974 vergangenen Zeit war der operative Eingriff das einzige Mittel um die Überlebenszeit zu erhöhen, oder in seltenen Fällen eine Gesundung herbeizuführen. 1974/75 wurden dann die Benzimidazole, darunter auch Mebendazol ((Methyl [5-benzoylbenzimidazol-2-carbamat]), für die Chemotherapie entdeckt (CAMPELL, W.C. et al., J. Parasitol. 61, 844-852, 1975; HEATH, D.D. et al., Parasitology 70, 273-285, 1975).

Bis heute stellt Mebendazol in der Regel das einzige Mittel dar, welches bei einer alveolären Echinokokkose eingesetzt wird. Die Prognosen bleiben jedoch weiterhin unbefriedigend, da diese Substanz in der Regel trotz täglicher Einnahme nur parasitostatisch wirkt und ihre Applikation nicht ganz unproblematisch ist. Hinzukommt, daß die Mebendazol - Behandlung keine völlige Ausheilung der Infektion bewirkt und nach Absetzen dieses Mittels das proliferative Wachstum der Parasitenstadien (Metacestoden) erneut einsetzen kann.

Letztendlich bleibt dann nur noch der Versuch einer Operation mit weiterhin unsicherer Prognose.

Benzimidazole bewirken eine Hemmung der Mikrotubuli-Synthese, indem sie an den dimeren Baustein, dem Tubulin, binden (H. VAN DEN BOSSCHE et al., In: Advances in pharmacology and chemotherapy. Vol. 19 [S. Garattini, A. Goldin, F. Hawkins, I. Kopin, Hrsg.], Academic Press New York, 67-128, 1982). Dabei ist die Bindungsaffinität nicht bei jedem Tubulin gleich hoch.

Von P.A. FRIEDMANN & E.G. PLATZER, Biochem. Biophys. Acta 630, 271-278, 1980, ist bekannt, daß z.B. eine 384-mal höhere Bindung von Mebendazol an embryonales Ascaris suum- Tubulin gegenüber Rinderhirn-Tubulin erfolgt. Mikrotubuli gehören zum zellulären Cytoskelett und haben neben Stützfunktion auch Anteil am Aufbau der Mitosespindel und am intrazellulären Transport. Folgen der Benzimidazol-Wirkung können Zellteilungsstörungen, Hemmung der Glucose-Aufnahme (H. VAN DEN BOSSCHE, In: Comparative biochemistry of parasites [H. van den Bossche, Hrsg.], Academic Press, New-York, 139-157; 1972) mit verstärktem Abbau endogenen Glykogens und Hemmung der ATP-Synthese (M.S. RAHMAN & C. BRYANT, Int. J. Parasitol. 7, 403-409, 1977) sein. Die dennoch nur parasitostatische Wirkung von Mebendazol auf Echinococcus multilocularis beruht unter anderem auf dem rein physikalischen Effekt der erschwerten Diffusion der Wirksubstanz durch das schwammartige Cystengewebe, mit zudem gelatinösen Lumeninhalt, im Vergleich zum Echinococcus granulosus (A. DIECKMANN, Dissertation, Fakultät II (Biol), Universität Hohenheim, 1987).

Mit der Mebendazol-Therapie der humanen Echinokokkose wurde vor ca. 13 Jahren begonnen. Sie ist auch heute noch mit mannigfaltigen Schwierigkeiten belastet. Aus ethischen Gründen und der hohen Mortalitätsrate konnten bisher keine Humanexperimente z.B. in Hinblick auf die minimal wirksame Mebendazolkonzentration durchgeführt werden. Außerdem besteht keine eindeutige Korrelation zwischen Dosis und Blutspiegel. Weitere Probleme treten mit dem Fehlen zuverlässiger Kriterien zur raschen Überprüfung von Erfolg oder Versagen einer Therapie und mangelnde Kenntnis über die Art und Ursache von Nebenwirkungen auf.

Allgemein wird heute bei inoperablem bzw. nicht-radikal operiertem und rezidivierendem Echinococcus multicularis eine Dauertherapie von 40-50 mg/kg/die angestrebt (R. AMMANN et al., In: Probleme der Echinokokkose unter Berücksichtigung parasitologischer und klinischer Aspekte [R. Bähr, Hrsg.], Huber Verlag Bern. Aktuel. Probl. Chir. Orthop. 23, 92-95, 1982; H. BIEDERMANN, ibidem, 98-99; U. JUNGE & P. FRIEDL, ibidem, 100-103; P. KERN & M. DIETRICH, ibidem, 104-105).

Es konnte bisher eine Verbesserung der Lebensqualität (H. BIEDERMANN 1982, loc. cit; U. JUNGE & P. FRIEDL, 1982, loc. cit.; P. KERN & M. DIETRICH 1982, loc. cit.), ein teilweiser Abfall des Echinococcus - AK-Titers (U. JUNGE & P. FRIEDL , 1982 loc. cit.), jedoch kein oder nur geringfügiger Rückgang der intrahepatischen Tumormasse (R. AMMANN et al., 1982, loc. cit.; P. KERN & M. DIETRICH, 1982, loc. cit.), erreicht werden. R. AMMANN et al., 1982, loc. cit., konnten bei 3 Patienten in operativ entnommenem Echinococcus - Material noch aktives Larvenmaterial nachweisen. So scheint weiterhin die ausschließlich parasitostatische Wirkung von Mebendazol bei einer Echinococcus - Infektion bestätigt zu sein. IFNγ wurde bisher hauptsächlich nur bei Virus-, Tumor- oder Autoimmunerkrankungen Eingesetzt oder zur Bekämpfung der diese Erkrankungen begleitenden Schmerzen (S. LEVIN, Isr. J. Med. Sci. 19, 955-958, 1983; K. OSTHER et al., Proceedings of the Second International TNO Meeting on the Biology of the Interferon System, 18.-22. April 1983, in: The Biology of the Interferon System 1983 [Edward De Maeyer & Huub Schellekens, Eds.] Elsevier Science Publishers B.V., 527-533, 1983).

Neben diesen Einsatzgebieten wurde vorgeschlagen, IFNγ auch bei intrazellulären Bakterieninfektionen anzuwenden (A.F. KIDERLEN et al., Eur. J. Immunol. 14, 964-967, 1984; J. MAUEL, Y. BUCHMÜLLER-ROUILLER, Eur. J. Immunol. 17, 203-208, 1987).

Darüberhinaus wurde bisher nur versucht, Infektionen durch parasitische Protozoen mit IFNγ zu therapieren (I.A. CLARK et al. J. Immunol. 139, 3493-3496, 1987; A.F. KIDERLEN, M.L. LOHMANN-MATTHES, Interdisciplinary conference on primary health care in the tropics, tropical diseases and zoonoses, 13.- 15. April 1987, Genf; S.G. REED, J. Immunol. 140 (12), 4342-4347, 1988; Y. SUZUKI et al., Science 240, 516-518, 1988). Die Effektivität dieser Therapieform war jedoch unsicher, teilweise sogar entmutigend.

Der vorliegenden Erfindung lag demnach die Aufgabe zugrunde, Arzneimittel bereitzustellen, welche, gegenüber dem Stand der Technik, wirkungsvoll zur Behandlung von Parasitosen, vorteilhafterweise von Infektionen durch Endoparasiten, besonders der Klasse Cestodes, vorzugsweise der Ordnung Cyclophyllidea, insbesondere der Gattung Echinococcus, beispielsweise durch Echinococcus multilocularis, eingesetzt werden können.

Erfindungsgemäß wird die Aufgabe dadurch gelöst, daß zur Behandlung der besagten Parasitosen eine Kombination aus IFNγ plus mindestens einem Anthelminthikum eingesetzt wird.

Aus den oben genannten Gründen war es nicht zu erwarten, daß die erfindungsgemäße Verabreichung einer Kombination aus IFNγ und mindestens einem Anthelminthikum eine wirksame Therapieform gegen die besagten Parasitosen darstellt.

Überraschenderweise wurde nun im Tierexperiment gefunden, daß die Verabreichung einer Kombination aus IFNγ und mindestens einem Anthelminthikum, letzteres vorzugsweise ausgewählt aus der Gruppe der chemisch definierten Anthelminthika, bei einer der gefährlichsten Parasitosen, mit Echinococcus multilocularis als Erreger, zu einem höheren Degenerations- bzw. Nekrosegrad der Cysten führte als bei einer Mebendazol - Monotherapie. Der Degenerations- bzw. Nekrosegrad war bei der erfindungsgemäßen Therapie zum Teil vollständig (100%). Weiterhin waren weder fertile noch sterile Cysten und entsprechend nur degenerierte Protoscoleces zu finden.

Zusätzlich konnte bei experimenteller oraler Infektion von Feldmäusen (Microtus arvalis), einem natürlichen Zwischenwirt, nach Verabreichung einer Kombination aus IFNγ und dem chemisch definierten Anthelminthikum Mebendazol eine schärfere Abgrenzung der Metacestoden-Cysten in der Leber gegen das umgebende Lebergewebe beobachtet werden als nach einer Mebendazol-Monotherapie.

Insbesondere waren die Leberzellen (Hepatocyten) in der unmittelbaren Umgebung der Herde weniger stark geschädigt als bei einer Mebendazol Monotherapie.

Eine Prüfung, ob damit auch das Auswachsen der Keimschichtsprosse verhindert oder zumindest reduziert wird, ist im Gange.

Dieser überraschende histologische Befung konnte durch die Messung der Serumspiegel des Leberenzyms GPT (Glutamat-Pyruvat-Transaminase) zusätzlich bestätigt werden. Dieses Enzym kommt fast ausschließlich in der Leber vor, wo es nur im Cytoplasma der Parenchymzellen vorhanden ist.

Je nach den Grad der Zellschädigung ist, bedingt durch eine unterschiedliche Freisetzungsrate, die Aktivität der GPT im Serum erhöht.

Der nach eigenen Intersuchungen ermittelte Normalwert der GPT-Aktivität im Serum von Feldmäusen (Microtus arvalis) beträgt durchschnittlich 72 U/l. Nach einer oralen Echinococcus multilocularis Infektion erhöhte sich 6 Wochen post infectionem die GPT-Aktivität auf durchschnittlich 120 U/l.

Mit einer Mebendazol Monotherapie über einen Zeitraum von 28 Tagen konnten die durchschnittliche GPT-Aktivität nur geringfügig auf 107 U/l gesenkt werden.

Überraschenderweise wurde nun gefunden, daß bei Kombination der vierwöchigen Mebendazol Therapie über einen Zeitraum von 14 Tagen mit IFNγ der GPT-Spiegel im Serum auf 78 U/l sank und sich damit nur geringfügig vom Normalwert (72 U/l) unterscheidet.

Demgegenüber bestätigte eine Monotherapie mit Mebendazol die ausschließlich parasitostatische Wirkung dieses Chemotherapeutikums. Es kam zwar zu umfassenden Schädigungen der Cysten, Protoscoleces und Keimschichtelementen, mit Kollaps- und Nekroseerscheinungen, doch waren Keimschichtsprosse weiterhin präsent. Positive Reimplantationsversuche unterstützen die Aussage, daß der Parasit regenerationsfähig bleibt.

Eine Monotherapie mit IFN-γ zeigte eine massive Zunahme des absoluten Cystengewichts (LCM= larval cyst mass) bis zur 6-fachen Masse gegenüber unbehandelten Tieren und wird auf immunsuppressive Eigenschaften dieser cytotoxischen Substanz zurückgeführt. Histologisch zeichneten sich die Cysten durch besonders häufige Sterilität, verdicktem Granulationswall und teilweise größeren Degenerationserscheinungen aus. Dennoch sind in der Regel eine Keimschicht sowie fertile Cysten vorhanden.

Der multiple Befall der Bauchhöhle, in einigen Fällen zusätzlich besonders am Diaphragma, läßt keinerlei Schluß auf eine Metastasierungshemmung durch IFN-γ zu.

Die besten Ergebnisse erzielte die Kombinationstherapie Mebendazol/IFN-γ. Es wurde hierbei eine LCM-Reduktionsrate von bis zu 99% erreicht.

Das für die erfindungsgemäße Kombination zu verwendende IFNγ kann über die bekannten Methoden der konventionellen Zellkulturen tierischer bzw. menschlicher Herkunft hergestellt werden, beispielsweise gemäß W.R. BENJAMIN et al., Proc. Natl. Acad. Sci. USA. 79, 5379-5383, 1982; Y.K. YIP et al., Proc. Natl. Acad. Sci. USA 78, 1601-1605, 1981; J.A.O'MALLEY, Methods Enzymol. 78, 540-545, 1981; Y.K.YIP et al., Proc. Natl. Acad. Sci. USA 79, 1820-1824, 1982, oder über die ebenfalls bekannte Technik der DNA-Rekombination, beispielsweise gemäß P.W. GRAY et al., Nature 295, 503-508, 1982; E. RINDERKNECHT et al., J. Biol. Chem. 259, 6790-6797; R. DEVOS et al., Nucl. Acids Res. 10, 2487-2501. 1982.

Bevorzugt wird für die erfindungsgemäße Kombination ein IFNγ, welches durch DNA-Rekombination nach den bekannten Methoden erhalten werden kann.

Für den Durchschnittsfachmann ist bekannt, daß natürliche allele Variationen individuumspezifisch vorkommen und sich durch eine oder mehrere unterschiedliche Aminosäuren oder durch unterschiedliche Nukleotide bzw. DNA-Sequenzen manifestieren. Derartige Variationen oder Mutationen, die auch durch die bekannten Methoden der DNA-Rekombination bzw. durch gezielte Mutagenese erzeugt werden können, wie sie beispielsweise von P.W. GRAY et al., 1982, loc. cit. und R. DEVOS et al., 1982, loc. cit. beschrieben werden, umfassen einfache oder mehrfache Substitutionen, Deletionen, Additionen, Insertionen oder Inversionen. Derartige IFNγs werden daher erfindungsgemäß mitumfaßt.

Aus immunologischen Gründen ist dem Fachmann bekannt, daß er beim Einsatz von biologisch aktiven, körpereigenen Wirkstoffen vorzugsweise auf speziesspezifische Wirkstoffe zurückgreifen wird. Für die erfindungsgemäße, speziesspezifische Verwendung von IFNγ wird demnach das aus dem jeweiligen, speziesspezifischen Geweben isolierte IFNγ bevorzugt oder die aus den speziesspezifischen Geweben oder Zellen isolierten Nukleinsäuren (RNA, DNA) zur Herstellung des jeweiligen IFNγ via DNA-Rekombination, insbesondere aber das dem jeweiligen, genuinem IFNγ identische Polypeptid mit dem bekannten biologischen Aktivitätsspektrum von IFNγ. Beispielsweise wird somit das für die erfindungsgemäße Verwendung am Menschen benutzte IFNγ ein human-IFNγ sein.

Für die erfindungsgemäße Anwendung von IFNγ kommen die dem Fachmann bekannten und gebräuchlichen pharmazeutischen bzw.galenischen Formulierungen für die orale oder parenterale Applikation in Betracht, vorzugsweise aber die für die parenterale Applikation, insbesondere für die intravenöse, intramuskuläre, subcutane, intracutane, intraartikuläre, intrathekale, intraperitoneale Infusion oder Injektion, wobei Dauerinfusionen oder intermittierende Infusionen mit den für den Fachmann zur Verfügung stehenden Pumpen miteingeschlossen sind.

Zur Herstellung einer gebrauchsfertigen Lösung für die erfindungsgemäße Verwendung von IFNγ stehen dem Fachmann die ihm zu diesem Zweck bekannten wässrigen Infusions- und Injektionslösungen zur Verfügung, gegebenenfalls zusammen mit den ihm bekannten Hilfs-, Träger - und/oder Stabilisierungsstoffen. Eine gebrauchsfertige Lösung für die erfindungsgemäße Verwendung ist beispielsweise auf die Weise herzustellen, in dem hochgereinigtes IFNγ in "Wasser für Injektionszwecke" oder in mit Phosphat gepufferter physiologischer Salinelösung (pH 7 bis 7,5), gegebenenfalls mit Tween und/oder Gelatine oder Human Albumin als Stabilisatoren supplementiert, vor der Applikation gelöst und in geeignete Gefäße (z.B. Ampullen, Beutel) steril abgefüllt wird.

Die zu verabreichende Menge an IFNγ für die erfindungsgemäße Verwendung orientiert sich an den für den Fachmann bekannten Dosierungen, an der Schwere der Erkrankung, an der Ansprechrate und an dem weiteren Verlauf der Erkrankung sowie an den Nebenwirkungen. Allgemein ist daher davon auszugehen, daß die Dosierung nach individuellen Kriterien zu erfolgen hat.

Die für die erfindungsgemäße Kombination zu verwendenden Anthelminthika, wovon mindestens eines davon als Bestandteil der erfindungsgemäßen Kombination vorliegt, sind vorteilhafterweise die bekannten chemisch definierten Anthelminthika, besonders diejenigen aus der Gruppe der Benzimidazole, insbesondere Mebendazol (Methyl[5-benzoylbenzimidazol-2-carbamat]). Die Substanz Mebendazol ist bekannt und beispielsweise unter dem Handelsnamen Vermox[R] oder Vermox[R] forte erhältlich (Fa. Janssen). Die Applikationsart und die Dosierung orientieren sich an den Therapieschemata, wie sie für die besagten Anthelminthika bekannt sind.

Die erfindungsgemäße Verwendung der Kombination IFNγ plus mindestens ein Anthelminthikum, vorteilhafterweise IFNγ plus mindestens ein Anthelminthikum aus der Gruppe der chemisch definierten Anthelminthika, besonders IFNγ plus mindestens ein Anthelminthikum aus der Gruppe der Benzimidazole, insbesondere IFNγ plus Mebendazol kann entweder durch gleichzeitige Gabe der beiden verschiedenen Wirkstoffklassen als auch durch konsekutive oder sequenzielle Applikation über die jeweils geeignete Route erfolgen. Vorteilhafterweise wird bei der erfindungsgemäßen Kombinationstherapie erst IFNγ und auschließend ein oder mehrere der beschriebenen Anthelminthika verabreicht.

Für eine gleichzeitige Anwendung von Mebendazol und IFNγ können beispielsweise solche Applikationsformen verwendet werden, die in Form von Liposomen herstellbar sind, beispielsweise gemäß der EP-A 213 523.

Legenden zu den Figuren

Fig. 1:     Versuchseinheit A, Rennmäuse; I= unbehandelt, II = Mebendazol, III = Mebendazol/IFNγ; F = Fertilität, S = Sterilität, D = Degeneration, N = Nekrose.

Fig. 2:     Versuchseinheit B, Rennmäuse; I = unbehandelt, II = Mebendazol, III = IFNγ; F, S, D und N wie bei Fig. 1.

Fig. 3:     Veränderung der Cystengewichte (LCM) bei Versuchseinheit A, Feldmäuse; o————o = ohne Therapie, o----o = Mebendazol, o....o = Mebendazol/IFNγ

Fig. 4:     Versuchseinheit A, Feldmäuse; I = unbehandelt, II =Mebendazol, III = Nebendazol/IFNγ; F, S, D und N wie bei Fig. 1.

Fig. 5:     Veränderung der Cystengewichte (LCM) bei Versuchseinheit B, Feldmäuse ; o————o = ohne Therapie, o----o = Mebendazol, o.....o = IFNγ.

Fig. 6:     Versuchseinheit B, Feldmäuse; I = unbehandelt, II = Mebendazol, III = IFNγ; F, S, D und N wie bei Fig. 1.

Die folgenden Beispiele sollen die Erfindung näher erläutern, sie aber in keiner Weise einschränken.

Beispiel 1 Befunde und Ergebnisse bei der Rennmaus Meriones unguiculatus als Zwischenwirt

Als Ausgangsmaterial dienten insgesamt 40 mit Metacestodenmaterial von E. multilocularis infizierte Rennmäuse, die gemäß Tabelle 1 in zwei Versuchseinheiten A und B unterteilt wurden.

Tabelle 1

|  | Versuchsgruppe | Therapiemodus | Anzahl Tiere |
|---|---|---|---|
| Versuchseinheit A | I | ohne | 6 |
| n=18 (8 männl., | II | Mebendazol | 6 |
| 10 weibl.) | III | Mebendazol plus IFNγ* | 6 |
| Versuchseinheits | I | ohne | 3 |
| n=22 (15 männl., | II | Mebendazol | 4 |
| 7 weibl. | III | IFNγ* | 15 |

*murines über DNA-Rekombination hergestelltes IFNγ

Die Auswahl des Geschlechts erfolgte willkürlich, das Alter betrug bei Versuchsbeginn zwischen 2 und 4 Monate. Die Versuchstiere wurden in einer Anzahl von 1 bis 6 in Makrolonkäfigen auf Sägemehl und Heu bei einem Tag-/Nacht-Rhythmus von 12 Stunden gehalten. Als Standarddiät diente Altromin[R]-Futter (Nr. 1314) und Leitungswasser ad libitum.

Für die Infektion der Tiere wurden zunächst Feldmäuse (Microtus arvalis) über die orale Route mit Eimaterial aus dem Darm eines natürlich befallenen Fuchses infiziert. Dieses Infektionsmaterial wurde anschließend nach der Methode von E. HINZ, Tropenmed. Parasitol. 23, 387-390, 1972, in 2-monatigem Abstand auf Meriones unguiculatus vegetativ weiter passagiert, indem pro Versuchstier o,4 ml einer Metacestoden-PBS (phosphat buffered solution) - Suspension intraperitoneal (ca. 50% gepacktes Material) appliziert wurde.

Die Gabe von Mebendazol erfolgte per os in der Weise, daß pulverisierte Tabletten (Vermox[R] forte, Fa. Janssen, Wirkstoffkonzentration 500 mg) dem ebenfalls pulverisiertem Futter zugemischt, mit einer Laborpresse (L 175; Amandus Kahl Nachf./Reinbek) zu Pellets verarbeitet und den Tieren ad libitum zur Verfügung gestellt wurden. In Anlehnung an die Erfahrungswerte von J. ECKERT et al., Schweiz. med. Wschr. 108, 1104-1112, 1978, war die Dosierung 500 ppm, entsprechend einer Tagesdosis von ca. 30-50 mg/kg Körpergewicht. Murines über DNA-Rekombination hergestelltes IFNγ (Genentech Inc. South San Francisco, Kalifornien, oder herstellbar nach P.W. GRAY, D.V. GOEDDEL, Proc. Natl. Acad. Sci. USA 80, 5842-5846, 1983) - in der Folge als IFNγ bezeichnet - lag in einer steril filtrierten Lösung (0.02M Tris, pH 7.5, 0.9% NaCl) mit einer IFNγ-Konzentration von 1,1 mg/ml (spez. Aktivität: 1-2 x $10^7$ U/mg Protein) vor und wurde vor der Applikation mit einer 2%igen Serumlösung (Rennmausserum/PBS) auf 50μg/ml verdünnt. Pro Behandlung wurden 0,2 ml dieser Lösung, entsprechend 10μg IFNγ (spez. Aktivität: 1-2 x $10^5$ U/mg Protein) intraperitoneal appliziert.

Der Beginn der Therapien (Monosubstanzen und Kombination) erfolgte ab dem sechsten Tag nach der Infektion (d.p.i., dies post infectionem).

Therapiedauer : Mebendazol als Dauertherapie bis zum Zeitpunkt der Tötung (Versuchseinheit A mittels Chloroform, Versucheinheit B mittels Genickbruch; 28., 35., und 42. d. p. i.), IFNγ jeden zweiten Tag, insgesamt 10 mal.

Als Kriterien zur Beurteilung der makroskopisch - pathoanatomischen Befunde wurden sowohl das absolute Cystengewicht (LCM = larval cyst mass) gegenüber dem Tötungszeitpunkt als auch die Lokalisation und der Habitus der Cystenkonglomerate gewählt. Um eine Reduktion bzw. Zunahme des absoluten Cystengewichtes der beiden Therapiegruppen der Versuchseinheiten A und B zu ermitteln, wurden die Cystengewichte der untherapierten Gruppe jeweils gleich 100% gesetzt.

Versuchseinheit A:

Bei den untherapierten Rennmäusen war mit zunehmender Infektionsdauer eine stetige und steile Zunahme der LCM zu beobachten.

Bei der Mebendazol-behandelten Tiergruppe war eine starke, langsam zunehmende Reduktion der LCM zu beobachten, während die Mebendazol/IFNγ-behandelte Gruppe sogar eine zunächst etwas höhere und schließlich gleiche Reduktion wie bei der Mebendazol-Gruppe zeigte.

Als Kriterien für die Beurteilung der mikroskopisch - pathohistologischen Befunde dienten im histologischen Schnitt der geschätzte prozentuelle Anteil fertiler, steriler, degenerierter und nekrotisierter Cysten, die Anzahl und der Reife- bzw. Vitalitätsgrad der Protoscoleces sowie cytohistopathologische Befunde.

Die Prozentzahlen von Fertilität, Sterilität und Degeneration ergeben zusammen 100%. Die Nekrose ist Teil der Degeneration, wurde aber aus Gründen der Übersichtlichkeit gesondert dargestellt.

Die untherapierten Tiere zeigten eine Abnahme fertiler Cysten vom 28. d.p.i. bis 42. d.p.i. von 60% auf 25%. Im Gegenzug nahm die Sterilität der Cysten zu, mit einem Maximum von 50% am 35. d.p.i.. Dagegen war der Anteil degenerierter Cysten geringer (Maximum von 30% an 42. d.p.i.), ohne Zeichen einer nekrotischen Zersetzung.

Die Mebendazol-therapierten Tiere wiesen keinerlei fertile und nur wenig sterile Cysten (Maximum von 25% am 35. d.p.i.) auf. Dagegen war der Anteil degenerierter Cysten sehr hoch (Maxima von 100% am 28. und 42. d.p.i.). Anteilig hatte die nekrotische Zersetzung am 35. d.p.i. ein Maximum von 60% erreicht. Zum Teil wurden beginnende lytische Vorgänge vom Zentrum der Cystenkomplexe aus beobachtet.

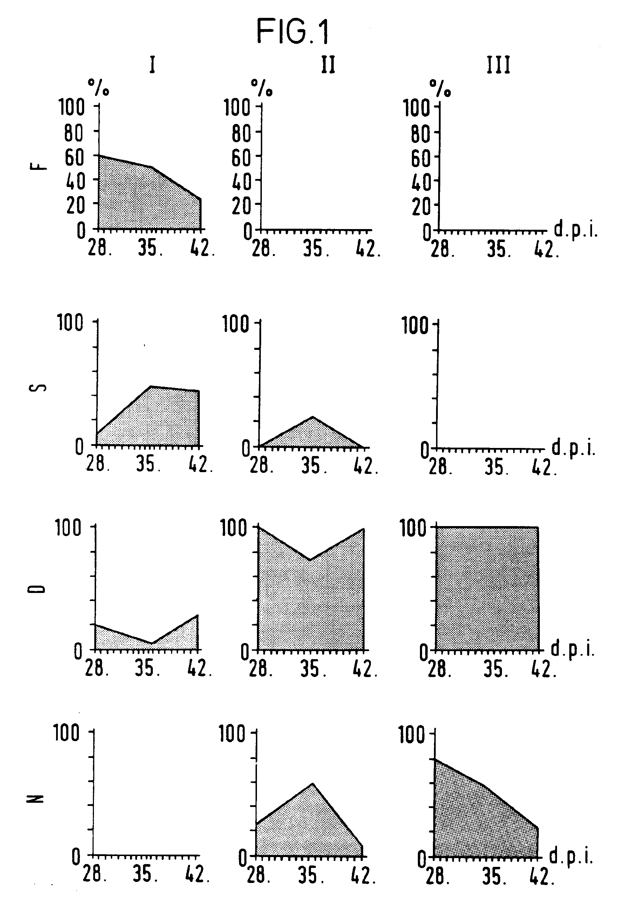

Die mit Mebendazol/IFNγ-therapierten Tiere hatten weder fertiles noch steriles Cystenmaterial. Die Degeneration lag hier konstant bei 100%, mit steil abnehmendem nekrotischen Anteil, von 80% am 28. d.p.i. auf 25% am 42. d.p.i.. Die Nekrose wurde hier grundsätzlich von lytischen Zersetzungen im Zentrum des Cystenkomplexes begleitet (Fig. 1).

In der untherapierten Gruppe traten einige, meist mittelreife, weniger junge und reife Protoscoleces auf. Jung bedeutet Kopfanlagen mit noch innerer Höhlung, mittelreif noch ohne Saugnäpfe und Hakenkranz und reif mit Saugnäpfen und Hakenkranz. Es wurden am 28. und 42. d.p.i. jedoch auch wenige degerierte Scoleces, erkennbar an ihrem amorphen Zellinhalt, beobachtet.

Sowohl die Mebendazol- als auch die Mebendazol/IFN-γ-therapierten Gruppen wiesen nur wenige, ausschließlich degenerierte Protoscoleces auf.

Versuchseinheit B:

Bei den untherapierten Rennmäusen war mit zunehmender Infektionsdauer zunächst ein leichter Anstieg, dann ein leichter Abfall des Cystengewichtes erkennbar.

Die Mebendazol-Gruppe zeigte eine geringfügig schwächere Reduktion der LCM als die gleiche Gruppe der Versuchseinheit A, war aber grundsätzlich vergleichbar. Die IFN-γ-Gruppe wies zunächst eine Reduktion der LCM um ca. 1/3 gegenüber dem untherapierten Kontrolltier auf, zeigte dann aber eine völlig unerwartete steile Zunahme um ca. das 6-fache.

Hinsichtlich Fertilität, Sterilität, Degeneration und Nekrose stimmten die untherapierten Tiere in der Tendenz weitgehend mit denen aus der Versuchseinheit A überein. Es wurde lediglich eine geringfügige nekrotische Zersetzung beobachtet.

Bei den Mebendazol-therapierten Tieren traten im Gegensatz zu Versuchseinheit A weder fertile noch sterile Cysten auf. Der Grad der Degeneration betrug 100%, mit steil zunehmendem Nekroseanteil.

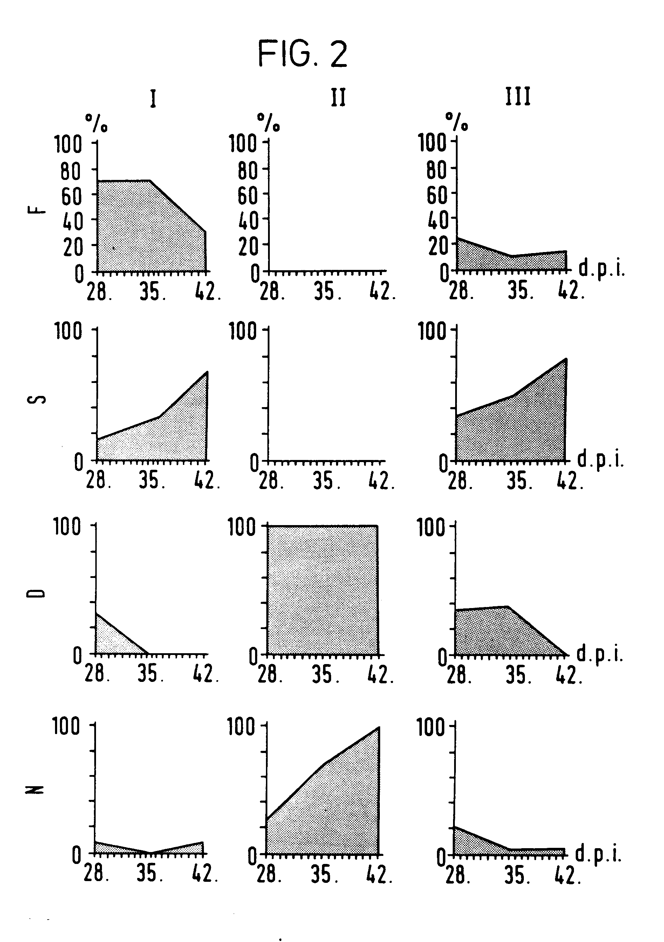

Die IFN-γ-therapierten Tiere wiesen nur geringgradig fertile Cysten auf, mit einem Maximum von 25% am 28. d.p.i. Der Sterilitätsgrad nahm dagegen steil zu (Maximum 80% am 42. d.p.i.). Die Degeneration fiel dagegen von knapp 40% auf 5% ab. Der Nekroseanteil war dabei gering (Fig. 2).

Betreffend Gesamtzahl, Reife- bzw. Vitalitätsgrad der Protoscoleces traten bei der untherapierten Gruppe einige, teils junge, teils mittelreife, jedoch wenig reife Protoscoleces auf. Degenerierte Kopfanlagen wurden nicht gefunden.

Die Mebendazol-Gruppe wies ebenfalls einige Protoscoleces auf, die jedoch sämtlich degeneriert waren.

In der IFN-γ-Gruppe wurden im 28-Tage-Stadium nur wenige junge bis mittelreife und degenerierte Protoscoleces gefunden. Im 35-Tage-Stadium war die Köpfchenanzahl noch weiter reduziert. Es fanden sich ausschließlich mittelreife bis reife, jedoch nur in einem Fall degenerierte Protoscoleces. Im 42-Tage-Stadium traten wenige, junge bis reife, aber keine degenerierten Scolexanlagen auf.

Beispiel 2: Befunde und Ergebnisse bei der Feldmaus Microtus arvalis als Zwischenwirt

Als Ausgangsmaterial wurden insgesamt 56 mit Metacestodenmaterial von E. multilocularis infizierte Feldmäuse verwendet. Das Metacestodenmaterial wurde nach oraler Erstinfektion nach der Methode von E. HINZ, Tropenmed. Parasitol. 23, 387-390, 1972, vegetativ weiterpassagiert. Analog zu Beispiel 1 wurden die Tiere in zwei Versuchseinheiten A und B (Tabelle 2) unterteilt.

## Tabelle 2

| | Versuchsgruppe | Therapiemodus | Anzahl Tiere |
|---|---|---|---|
| Versuchseinheit A | I | ohne | 10 |
| n = 30 (17 männl., | II | Mebendazol | 10 |
| 13 weibl.) | III | Mebendazol plus IFNγ* | 10 |
| Versuchseinheit B | I | ohne | 5 |
| n = 26 (9 männl., | II | Mebendazol | 4 |
| 17 weibl.) | III | IFNγ* | 17 |

* murines über DNA-Rekombination hergestelltes IFNγ

Die Auswahl des Geschlechts, das Alter der Tiere, die Tierhaltung, die Ernährung, die Gabe der Metacestoden-Suspension für die vegetative Weiterpassage, die Infektionsdosis und die Applikationsweise von Mebendazol entsprachen den Angaben in Beispiel 1. In Analogie zu Beispiel 1 erfolgte die Gabe der IFNγ-Lösung mit den Ausnahmen, daß Feldmausserum/PBS zur Verdünnung verwendet wurde und daß pro Behandlung 0.1 ml der fertigen Lösung, entsprechend 5μg IFNγ (spez. Aktivität 0.5 - 1 x $10^5$ U/mg Protein), appliziert wurden.

Der Beginn der Therapien (Monosubstanzen und Kombinationen) erfolgte ab 21. d.p.i.. Dieser gegenüber Beispiel 1 verzögerte Therapiebeginn liegt in dem langsameren Wachstum des Metacestodenmaterials in Microtus arvalis als natürlicher Zwischenwirt begründet.

Therapiedauer: Mebendazol als Dauertherapie bis zum Zeitpunkt der Tötung (Versuchseinheit A mittels Chloroform, Versuchseinheit B mittels Genickbruch; 41., 48., 55., 62. und 69. d.p.i.), IFNγ wie unter Beispiel 1 angegeben.

Für die Reimplantationsversuche wurden von 2 Feldmäusen (die eine mit Monotherapie Mebendazol, die andere mit Kombinationstherapie Mebendazol/IFNγ) bei der Sektion am 62. d.p.i. zusätzlich je ein Cystenstück isoliert, in PBS-Antibiotikum-Lösung eingelegt und über Nacht kühlgestellt. Nach Zerteilen des jeweiligen Cystenstückes in 2 nahezu gleiche Teile, erhielten diese je 2 männliche Rennmäuse nach der Methode von E. GRIMMINGER, Diplomarbeit, Universität Hohenheim, 1984, reimplantiert. Die Beurteilungskriterien der makroskopisch - pathoanatomischen Befunde waren wie in Beispiel 1 angegeben.

Versuchseinheit A:

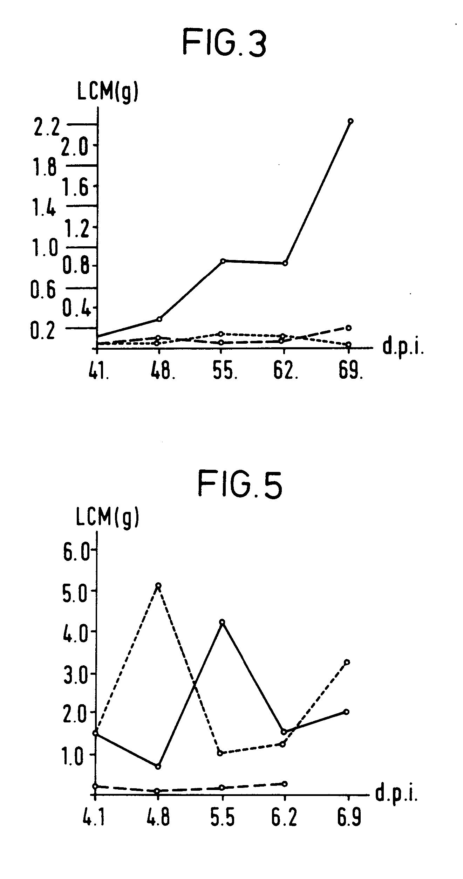

Die Cystengewichte der unbehandelten Feldmäuse stiegen im Gegensatz zu den Rennmäusen stufenförmig an, wobei zwischen dem 55. und 62. d.p.i. scheinbar ein Stillstand erreicht worden ist, um danach aber wieder steil in die Höhe zu schnellen. Im Vergleich dazu war bei den Mebendazol-therapierten Tieren zunächst eine geringfügige Zunahme der LCM, dann aber eine relativ starke Reduktion, mit Höhepunkt am 55. d.p.i. und wieder leichtem Abfall zum 69. d.p.i. hin, zu beobachten. Die mit Mebendazol IFNγ kombiniert behandelten Feldmäuse zeigten am 41. d.p.i. eine Reduktion um ca. 3/4 gegenüber den untherapierten Tieren, um sich dann stetig bis zu einem Maximum von 99% am 69. d.p.i. fortzuentwickeln (Fig.3).

Die Cysten aller 3 Versuchsgruppen waren zumeist über den ganzen Bauchraum verteilt, mit Ausnahme der Pleuralhöhle. In 3 Fällen fanden sich bei der untherapierten Gruppe mit der inneren Bauchhaut verwachsene Cysten.

Bei der Kombinationsgruppe Mebendazol/IFNγ war im Gegensatz zu den anderen beiden Versuchsgruppen kein Leberbefall zu beobachten.

Hinsichtlich Fertilität, Sterilität, Degeneration und Nekrose lag bei der untherapierten Gruppe der Fertilitätsgrad am 41. und 69. d.p.i. bei 100% und sank dazwischen nur um knapp 20% ab. Dementsprechend niedrig lagen Sterilitäts- und Degenerationsgrad.

Die Mebendazol-therapierte Gruppe zeigte keinerlei Fertilität und nur am 41. d.p.i. eine 15%-ige Sterilität. Die Degeneration lag entsprechend hoch, mit fast deckungsgleichem Nekroseanteil.

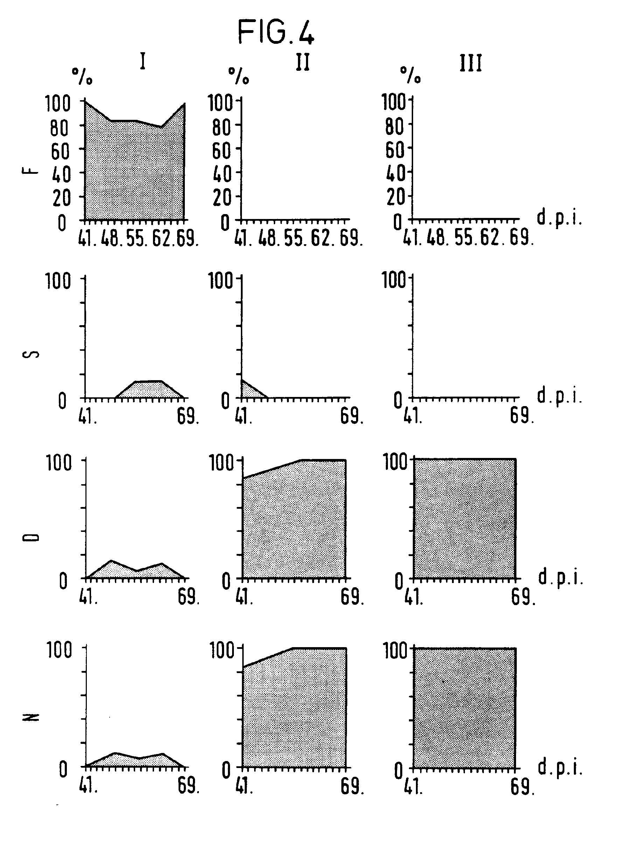

In der Mebendazol-/IFNγ-therapierten Gruppe konnten weder fertile noch sterile Cysten nachgewiesen werden. Degeneration und Nekrose lagen konstant bei 100% (Fig. 4).

Betreffend Gesamtzahl, Reife- bzw. Vitalitätsgrad der Protoscoleces zeigten die untherapierten Tiere durchgehend viele, meist reife, wenig junge und mittelreife und selten degenerierte Kopfanlagen.

Bei den Mebendazol- und Mebendazol-/IFNγ-therapierten Tieren traten dagegen nur vereinzelt Protoscoleces auf, die aber alle degeneriert waren.

Versuchseinheit B:

Die untherapierte Gruppe zeigte einen leicht aufsteigenden, zickzackförmigen Verlauf ihrer Cystengewichte, mit einem Maximum am 55. d.p.i..

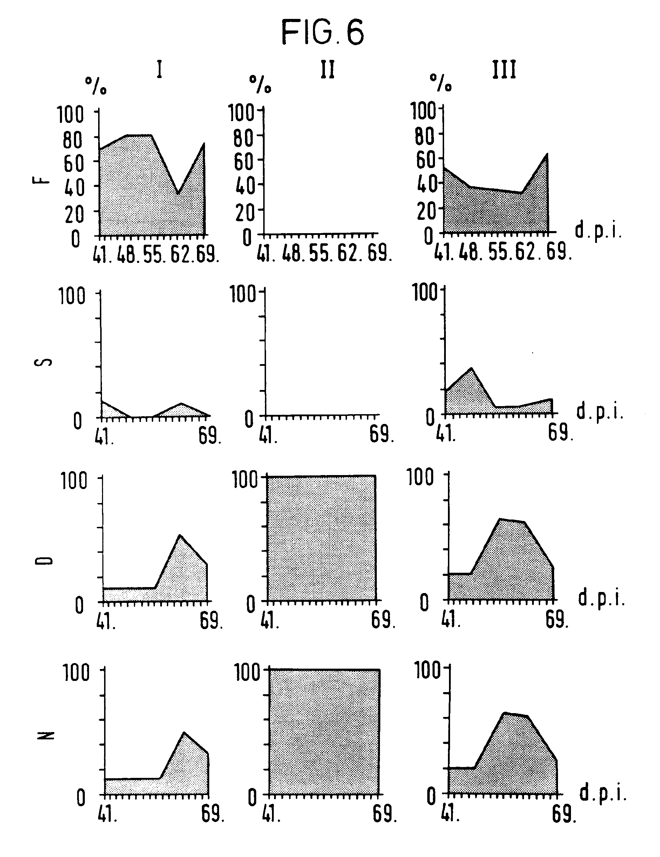

Die Mebendazol-behandelte Gruppe wies eine relativ starke Reduktion der LCM mit einem Maximum am 55. d.p.i. auf. Die IFNγ-therapierte Gruppe zeigte dagegen ein völlig konzeptloses Bild. Der Zickzackverlauf ähnelte dem der untherapierten Gruppe, das LCM-Maximum war hier jedoch auf den 48. d.p.i. vorverschoben. Analog zu Beispiel 1 kommt es auch hier unter IFNγ-Therapie zunächst zu einem massiven Anstieg der LCM (um ca. das 6-fache), um dann aber wieder drastisch abzusinken und erst ab dem 69. d.p.i. erneut stark zuzunehmen (Fig. 5).

Hinsichtlich Fertilität, Sterilität, Degeneration und Nekrose wurde bei der untherapierten Gruppe der höchste Fertilitätsgrad am 48. und 55. d.p.i. mit 90% erreicht. Ein tiefer Einbruch mit nur 30% Fertilität erschien am 62. d.p.i. der jedoch am 69. d.p.i. mit einem 70%-igen Fertilitätsgrad wieder behoben wurde. Sterilität trat kaum auf, dagegen verliefen Degeneration bzw. Nekrose (hier deckungsgleich, d.h. das degenerierte Gewebe ist vollständig nekrotisiert) negativ synchron zum Fertilitätsgrad.

Die Mebendazol-therapierte Gruppe zeichnete sich ausschließlich durch eine 100%-ige Degeneration bzw. Nekrose (ebenfalls deckungsgleich) aus.

Die IFNγ-therapierte Gruppe schließlich zeigte eine mäßige Fertilität zwischen 30 und 60%. Der Sterilitätsgrad lag am 48. d.p.i. mit knapp 40% am höchsten und sank dann unter 10% ab. Degeneration und Nekrose (wiederum deckungsgleich) nahmen vom 48. auf den 55. d.p.i. von gut 20% auf 65% steil zu und fielen zum 69. d.p.i. wieder auf 30% zurück (Fig. 6).

Betreffend Gesamtzahl, Reife- bzw. Vitalitätsgrad der Protoscoleces zeigten die untherapierten Tiere einige bis viele, meist reife, wenig junge und mittelreife, jedoch auch degenerierte Protocoleces.

Bei den Mebendazol-therapierten Tieren traten einige, jedoch degenerierte Kopfanlagen auf. Die IFNγ-therapierten Tiere wiesen bis zum 55. d.p.i. durchschnittlich einige, meist junge oder reife, weniger mittelreife und ab und zu wenige degenerierte Protoscoleces auf. Ab dem 62 d.p.i. erschienen viele, meist reife, weniger junge und noch weniger mittelreife und selten degenerierte Protoscoleces.

## Patentansprüche

**Patentansprüche für folgende Vertragsstaaten : AT, BE, CH, DE, DK, FR, GB, IT, LI, LU, NL, SE**

1. Arzneimittelkombination auf der Basis von mindestens einem Anthelminthikum, dadurch gekennzeichnet, daß sie als zusätzlichen Wirkstoff Interferon-gamma enthält.

2. Arzneimittelkombination nach Anspruch 1, dadurch gekennzeichnet, daß ein darin enthaltenes Anthelminthikum ausgewählt ist aus der Gruppe der chemisch definierten Anthelminthika.

3. Arzneimittelkombination nach Anspruch 2, dadurch gekennzeichnet, daß ein darin enthaltenes Anthelminthikum ausgewählt ist aus der Gruppe der Benzimidazole.

4. Arzneimittelkombination nach Anspruch 3, dadurch gekennzeichnet, daß ein darin enthaltenes Anthelminthikum Mebendazol ist.

5. Arzneimittelkombination nach einem der Ansprüche 1 bis 4, dadurch gekennzeichnet, daß das darin enthaltene IFNγ speziesspezifisch ist und aus natürlichen Zellen entstammt oder über DNA-Rekombination hergestellt worden ist.

6. Arzneimittelkombination nach Ansprüchen 1 bis 5 zur Behandlung von Helminthiasen bei Menschen und Säugetieren.

7. Arzneimittelkombination nach Anspruch 6 zur Behandlung von durch Cestoden verursachten Infektionen.

8. Arzneimittelkombination nach Anspruch 7 zur Behandlung von durch Echinococcus multilocularis verursachte Infektionen.

9. Verwendung von IFNγ und mindestens einem Anthelminthikum zur Herstellung von Arzneimittelkombinationen nach einem der Ansprüche 1 bis 8.

10. Arzneimittelpackung, bestehend aus mindestens einem Anthelminthikum nach den Ansprüchen 2 bis 4 und einem Interferon gamma.

**Patentansprüche für folgende Vertragsstaaten : ES, GR**

1. Verfahren zur Herstellung einer Arzneimittelkombination auf der Basis von mindestens einem Anthelminthikum, dadurch gekennzeichnet, daß dieser als zusätzlicher Wirkstoff Interferon-gamma zugesetzt wird.

2. Verfahren nach Anspruch 1, dadurch gekennzeichnet, daß ein in der Kombination enthaltenes Anthelminthikum ausgewählt ist aus der Gruppe der chemisch definierten Anthelminthika.

3. Verfahren nach Anspruch 2, dadurch gekennzeichnet, daß ein in der Kombination enthaltenes Anthelminthikum ausgewählt ist aus der Gruppe der Benzimidazole.

4. Verfahren nach Anspruch 3, dadurch gekennzeichnet, daß ein in der Kombination enthaltenes Anthelminthikum Mebendazol ist.

5. Verfahren nach einem der Ansprüche 1 bis 4, dadurch gekennzeichnet, daß das in der Kombination enthaltene IFNγ speziesspezifisch ist und aus natürlichen Zellen entstammt oder über DNA-Rekombination hergestellt worden ist.

6. Verfahren zur Herstellung einer Arzneimittelpackung, dadurch gekennzeichnet, daß diese aus mindestens einem Anthelminthikum nach den Ansprüchen 2 bis 4 und einem Interferon gamma zusammengesetzt wird.

**Claims**

**Claims for the following Contracting States : AT, BE, CH, DE, DK, FR, GB, IT, LI, LU, NL, SE**

1. Pharmaceutical combination based on at least one anthelminthic, characterised in that it contains interferon-gamma as an additional active substance.

2. Pharmaceutical combination according to claim 1, characterised in that the anthelminthic contained therein is selected from the group of chemically defined anthelminthics.

3. Pharmaceutical combination according to claim 2, characterised in that an anthelminthic contained therein is selected from the group of benzimidazoles.

4. Pharmaceutical combination according to claim 3, characterised in that an anthelminthic contained therein is mebendazole.

5. Pharmaceutical combination according to one of claims 1 to 4, characterised in that the IFNγ contained therein is species-specific and originates from natural cells or has been prepared by DNA recombination.

6. Pharmaceutical combination according to claims 1 to 5 for treating helminthiases in humans and mammals.

**7.** Pharmaceutical combination according to claim 6 for treating infections caused by cestodes.

**8.** Pharmaceutical combination according to claim 7 for treating infections caused by <u>Echinococcus</u> <u>multilocularis</u>.

**9.** Use of IFN$\gamma$ and at least one anthelminthic for preparing pharmaceutical combinations according to one of claims 1 to 8.

**10.** Pharmaceutical package consisting of at least one anthelminthic according to claims 2 to 4 and an interferon gamma.

**Claims for the following Contracting States : ES, GR**

**1.** Process for preparing a pharmaceutical combination based on at least one anthelminthic, characterised in that it contains interferon-gamma as an additional active substance.

**2.** Process according to claim 1, characterised in that an anthelminthic contained in the combination is selected from the group of chemically defined anthelminthics.

**3.** Process according to claim 2, characterised in that an anthelminthic contained in the combination is selected from the group of benzimidazoles.

**4.** Process according to claim 3, characterised in that an anthelminthic contained in the combination is mebendazole.

**5.** Process according to one of claims 1 to 4, characterised in that the IFN$\gamma$ contained in the combination is species-specific and originates from natural cells or has been prepared by DNA recombination.

**6.** Process for preparing a pharmaceutical package, characterised in that it is made up of at least one anthelminthic according to claims 2 to 4 and an interferon gamma.

**Revendications**

**Revendications pour les Etats contractants suivants : AT, BE, CH, DE, DK, FR, GB, IT, LI, LU, NL, SE**

**1.** Combinaison médicamenteuse à base d'au moins un antihelminthique, caractérisée en ce qu'elle contient un interféron gamma en tant que principe actif supplémentaire.

**2.** Combinaison médicamenteuse selon la revendication 1, caractérisée en ce qu'un antihelminthique qu'elle contient est choisi dans le groupe des antihelminthiques définis du point de vue chimique.

**3.** Combinaison médicamenteuse selon la revendication 2, caractérisée en ce qu'un antihelminthique qu'elle contient est choisi dans le groupe des benzimidazoles.

**4.** Combinaison médicamenteuse selon la revendication 3, caractérisée en ce qu'un antihelminthique qu'elle contient est le mébendazole.

**5.** Combinaison médicamenteuse selon l'une des revendications 1 à 4, caractérisée en ce que l'IFN $\gamma$ qu'elle contient est à spécificité d'espèce et provient de cellules naturelles ou a été préparé par recombinaison d'ADN.

**6.** Combinaison médicamenteuse selon les revendications 1 à 5 pour le traitement des helminthiases chez l'homme et les mammifères.

**7.** Combinaison médicamenteuse selon la revendication 6 pour le traitement des infections provoquées par les cestodes.

8. Combinaison médicamenteuse selon la revendication 7 pour le traitement des infections provoquées par <u>Echinococcus</u> <u>multilocularis.</u>

9. Utilisation d'IFN γ et d'au moins un antihelminthique pour la préparation de combinaisons médicamenteuses selon l'une des revendications 1 à 8.

10. Présentation médicamenteuse consistant en au moins un antihelminthique selon les revendications 2 à 4 et en un interféron gamma.

**Revendications pour les Etats contractants suivants : ES, GR**

1. Procédé de préparation d'une combinaison médicamenteuse à base d'au moins un antihelminthique, caractérisé en ce qu'on ajoute à celle-ci un interféron gamma en tant que principe actif supplémentaire.

2. Procédé selon la revendication 1, caractérisé en ce qu'un antihelminthique contenu dans la combinaison est choisi dans le groupe des antihelminthiques définis du point de vue chimique.

3. Procédé selon la revendication 2, caractérisé en ce qu'un antihelminthique contenu dans la combinaison est choisi dans le groupe des benzimidazoles.

4. Procédé selon la revendication 3, caractérisé en ce qu'un antihelminthique contenu dans la combinaison est le mébendazole.

5. Procédé selon l'une des revendications 1 à 4, caractérisé en ce que l'IFN γ contenu dans la combinaison est à spécificité d'espèce et provient de cellules naturelles ou a été préparé par recombinaison d'ADN.

6. Procédé de préparation d'une présentation médicamenteuse, caractérisé en ce que celle-ci consiste en au moins un antihelminthique selon les revendications 2 à 4 et en un interféron gamma.

# FIG.1

# FIG. 2

# FIG.3

# FIG.5

# FIG.4

# FIG. 6